(19) Europäisches Patentamt
European Patent Office
Office européen des brevets

(11) Veröffentlichungsnummer: **0 031 941**
**A1**

# (12) EUROPÄISCHE PATENTANMELDUNG

(21) Anmeldenummer: **80108105.0**

(22) Anmeldetag: **22.12.80**

(51) Int. Cl.³: **C 07 H 17/02**
C 12 Q 1/40, C 12 P 19/04
C 12 P 19/26

(30) Priorität: **05.01.80 DE 3000292**

(43) Veröffentlichungstag der Anmeldung:
**15.07.81 Patentblatt 81/28**

(84) Benannte Vertragsstaaten:
**AT BE CH DE FR GB IT LI NL SE**

(71) Anmelder: **BEHRINGWERKE AKTIENGESELLSCHAFT**
**Postfach 1140**
**D-3550 Marburg/Lahn(DE)**

(72) Erfinder: **Wallenfels, Kurt, Prof. Dr.**
**Vordere Steige 7**
**D-7800 Freiburg(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) **Indoxylmaltodextrine, Verfahren zu ihrer Herstellung und ihre Verwendung.**

(57) Indoxylmaltodextrine, der Formel:

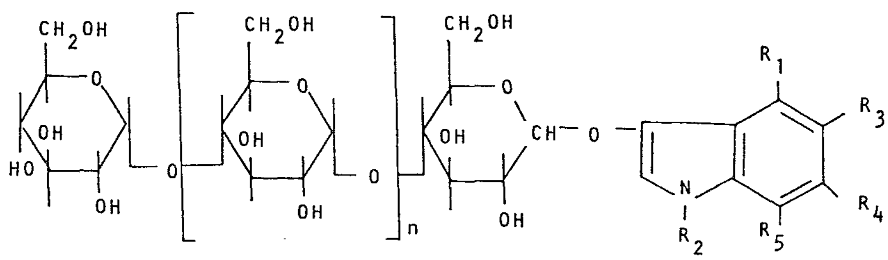

in der n eine ganze Zahl von 1 bis 9, $R_1$, $R_3$, $R_4$ and $R_5$ Halogen, Alkyl oder Alkoxyl mit 1 - 5 Kohlenstoffatomen, Phenyl, substituiertes Phenyl, Phenoxy, substituiertes Phenoxy, Nitro oder Wasserstoff, $R_2$ = H, niedriges Alkyl oder niedriges Alkoxy mit 1 - 3 C-Atomen in der Alkylkette ist und die heterosidische Glucosidbindung α-oder β-Konfiguartion aufweist, ein enzymatisches Verfahren zu deren Herstellung und deren Verwendung als Substrat für den analytischen Nachweis und die Bestimmung von Amylasen beschrieben sowie Schnelldiagnostika, die solche Indoxylmaltodextrine enthalten.

EP 0 031 941 A1

BEHRINGWERKE AKTIENGESELLSCHAFT HOE 80/B 001 Dr.HA/Bl

# Indoxylmaltodextrine, Verfahren zu ihrer Herstellung und ihre Verwendung

Die Erfindung betrifft neue Indoxylmaltodextrine, deren enzymatische Synthese und deren Verwendung als Substrat für den analytischen Nachweis und die Bestimmung von Amylasen sowie Schnelldiagnostika, die Indoxylmaltodextrine enthalten.

Die Amylase-Bestimmung sowohl im Blutserum als auch im Harn hat in der klinisch-chemischen Diagnostik eine große Bedeutung für die Erkennung von Pankreas-Erkrankungen.

Es sind eine Reihe verschiedener Methoden in der Literatur beschrieben worden, die die quantitative Bestimmung erlauben. Beispiel hierfür ist die DE-OS 27 31 421, wonach nitroaromatische Glycoside als Substrat für die Bestimmung der Amylase eingesetzt werden. Die Reaktionsprodukte dieser Substrate sind wasserlöslich. Die im wässrigen System ablaufenden Bestimmungsverfahren haben ihre Bedeutung in klinischen Laboratorien. Sie sind jedoch alle arbeitsaufwendig, benötigen teure Meßinstrumente für die quantitative Erfassung des Ergebnisses und erfordern technisch ausgebildetes Personal. In der Praxis muß jedoch oftmals schnell und am Krankenbett abgeklärt werden, ob im speziellen Fall eine Pankreas-Erkrankung vorliegt. Mit Hilfe von Schnelldiagnostika, wie z.B. Teststreifen, lassen sich heute zahlreiche diagnostisch interessante Körperbestandteile schnell und einfach bestimmen. Diese oftmals als Screening eingesetzten Analysenmethoden haben daher eine weite Verbreitung gefunden. Für die Bestimmung von Amylase besteht ebenfalls ein Bedarf an einem Schnelldiagnostikum. Die oben erwähnten Substrate sind hierfür jedoch nicht geeignet.

Da die Nachweismethoden in nahezu allen Fällen in einem wässrigen Medium ablaufen und die Reaktionsprodukte wasserlöslich sind, müssen bei der Herstellung von Teststreifen besondere Vorkehrungen getroffen werden, um ein Auswaschen bzw. Ausbluten von Reaktionskomponenten zu verhindern.

In der Literatur (J.Med.Chem., Band 7, S. 574, 1964) sind bereits chromogene Verbindungen beschrieben, die zu wasserunlöslichen Farbstoffen, Indigoderivaten, umgesetzt werden. So wurde beispielsweise 5-Brom-4-chlorindoxyl-ß-D-glucosid zum histochemischen Nachweis der ß-Glucosidase beschrieben. Diese bekannten Verbindungen sind für die Amylase-Bestimmung jedoch ungeeignet.

Es war daher das Ziel und die Aufgabe der Erfindung, einen schnell und einfach durchzuführenden Test, ein Schnelldiagnostikum auf alpha-Amylase zu entwickeln, in dem das Reaktionsprodukt des Substrats für die Amylase wenig wasserlöslich ist und daher nicht ausblutet. Zunächst liegt der Erfindung die Aufgabe zugrunde, ein solches Substrat für die Amylasebestimmung bereitzustellen.

Es wurde nun überraschenderweise festgestellt, daß Indoxylmaltodextrine die gewünschten Eigenschaften besitzen.

Gegenstand der Erfindung sind Indoxylmatodextrine der allgemeinen Formel :

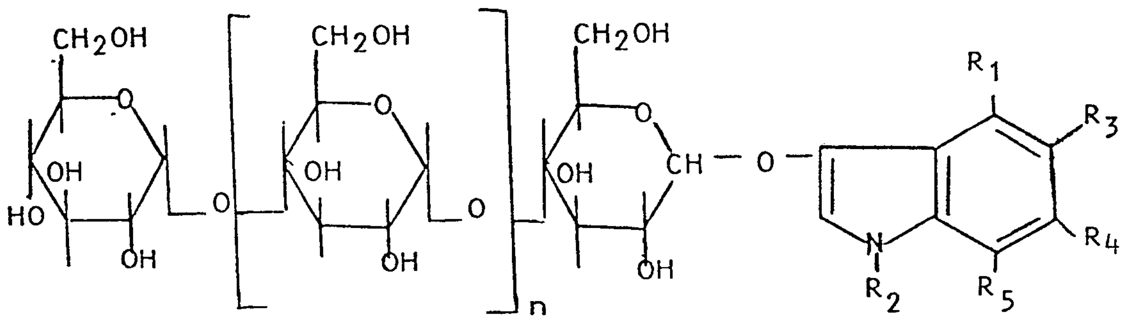

in der n eine ganze Zahl von 1 bis 9, $R_1$, $R_3$, $R_4$ und $R_5$ Halogen, Alkyl oder Alkoxyl mit 1 - 5 Kohlenstoffatomen, Phenyl, substituiertes Phenyl, Phenoxy, substituiertes Phenoxy, Nitro oder Wasserstoff, $R_2$= H, niedriges Alkyl oder niedriges Alkoxy mit 1 - 3 C-Atomen in der Alkylkette ist und die heterosidische Glucosidbindung α- oder ß-Konfiguration aufweist.

Die Verbindungen sind geeignete Substrate für Amylasen. Mit ihnen läßt sich die Amylaseaktivität mit großer Empfindlichkeit in einfacher Weise kolorimetrisch bestimmen und auch für Schnelldiagnostika einsetzen.

Insbesondere haben sich Indoxylmaltodextrine mit n = 2 - 4, d.h. mit 4 - 6 Glucoseeinheiten aufgrund ihrer schnellen Umsatzrate als vorteilhaft erwiesen.

Bestimmte Amylasen bauen nur zum Indoxylglucosid bzw. -maltosid ab. Deshalb hat es sich als zweckmäßig erwiesen, bei diesen Amylaseenzymen bei der erfindungsgemäßen Bestimmung der Amylaseaktivität eine α- bzw. ß-Glucosidase als Hilfsenzym mit zu verwenden, die dann die von den erfindungsgemäßen Indoxylmaltodextrinen mit

3 - 12 Glucoseeinheiten durch die Amylase abgespaltenen Indoxylglucoside oder Indoxylmaltoside schnell zum Aglukon weiterspalten können. Bei genügender Menge an diesem α- bzw. β-Glucosidase-Hilfsenzym im Ansatz werden diese Spaltprodukte in statu nascendi sofort durch das Hilfsenzym weitergespalten.

Das freigesetzte Indoxyl oxidiert in Gegenwart von Sauerstoff oder anderen Oxidationsmitteln zum Indigo, dessen Konzentration bzw. Farbintensität ein Maß für die Amylaseaktivität darstellt. Als Oxidationsmittel sind Eisen(III)-Verbindungen oder andere Metallsalze höherer Wertigkeit, die in der Lage sind, die Oxidation zum Indigo-Farbstoff zu bewerkstelligen, geeignet. Die Oxidation in Gegenwart von Sauerstoff wird am besten mit einer elektronenübertragenden Verbindung, wie z.B. Tetramethyl-p-phenylendiamin oder einem Enzym durchgeführt. Eine weitere Möglichkeit zur Oxidation des Indoxyls besteht in der Verwendung von Wasserstoffperoxid in Gegenwart eines Katalysators, wie Peroxidase, Katalase oder Metallsalzen.

Die Reaktionsgleichungen einer erfindungsgemäßen Amylase-Bestimmung lassen sich folgendermaßen beispielhaft darstellen:

1. Indoxylmaltotetraosid + $H_2O$ $\xrightarrow{\text{Amylase}}$ Indoxylglucosid + Maltotriose

2. Indoxylglucosid + $H_2O$ $\xrightarrow{\text{Glucosidase}}$ Indoxyl + Glucose

3. 2 Indoxyl $\xrightarrow{\text{Oxidation}}$ Indigo

Die für die Durchführung der erfindungsgemäßen Bestimmung von Amylase verwendeten Indoxyl-α-D-maltodextrine mit der Indoxylgruppe am reduzierenden Glucoserest des Maltodextrins mit 3 - 12 Glucoseeinheiten in der Kette sind neue Substanzen. Sie können erfindungsgemäß durch eine enzymatische Transglukanosilierung, ausgehend vom Indoxylglucosid, hergestellt werden. Hierbei ist das Ausgangsmaterial das α-Cyclodextrin und Indoxyl-α- oder ß-D-Glukosid mit einem Glucose-Rest. Die Überführung in die Indoxylmaltodextrine, im wesentlichen der Kettenlängen bis zu 12 Glukoseeinheiten, erfolgt durch Vermischen der Reaktionskomponenten in wässriger Lösung in Gegenwart einer Transglukanosylase, vorzugsweise derjenigen, die aus Klebsiella pneumoniae gewonnen und von BENDER, Arch. Mikrobiol., Band 111 (1977), 271-282, beschrieben wurde. Es kann aber auch eine andere Transglykanosylase, z.B. diejenige aus B.macerans für die Synthesereaktion eingesetzt werden oder auch andere Enzyme mit vergleichbarer Spezifität, z.B. die D-Transglykosidase aus Kartoffeln oder anderen Pflanzen, d.h. solche, welche Glukanosylreste von linearen oder cyclischen Dextrinen auf Akzeptoren übertragen können, wobei dann die Akzeptoren α- oder ß-Glucoside des Indoxyls sind. Ein weiterer Gegenstand der Erfindung ist ein Verfahren zur Herstellung von Indoxyl-α- oder ß-maltodextrinen, dadurch gekennzeichnet, daß man eine wässrige Lösung von α-, ß-, oder γ-Cyclodextrin und ein die Indoxylgruppe enthaltendes α- oder ß-D-Glucosid in Gegenwart einer Transglucanosylase reagieren läßt, worauf man die synthetisierten Indoxyl-α- bzw. ß-Maltodextrine in an sich bekannter Weise isoliert.

Die Synthese ausgehend von α-Maltosylfluorid und α-Amylase als transferierendes Enzym macht von folgendem Gebrauch:
α-Maltosylfluorid, dargestellt nach GENGHOF et al., Carbohydr. Res. 61 (1978) 291, wird von α-Amylasen, insbesondere von B. subtilis oder Schweinepankreas unter vorwiegendem Transfer von Maltosylresten auf Maltosylfluorid selbst gespalten, so daß Maltodextrine mit 3 - 6 Glucoseeinheiten entstehen (OKADA et al., Carbohydr. Res. 71 (1979), 287). Benützt man ein Heteroglucosid wie zum Beispiel Indoxylglucosid als Maltoseakzeptor, entstehen die entsprechenden Indoxylmaltodextrine mit 3 - 6 Glucoseeinheiten in der α-1,4-verknüpften Kette. Sie lassen sich in an sich bekannter Weise zum Beispiel durch Chromatographie in die einzelnen Komponenten auftrennen. α-Maltosylfluorid und α-Amylase werden dabei in Konzentrationen von ca. 5,5 mM bzw. 10 - 100 µg/ml in ca. 0,025 M Acetatpuffer von ca. pH 5,6 mit Indoxylglucosid (ca. 10 m M) inkubiert. Nach 1 - 12 Stunden kann aufgearbeitet werden.

Wenn es auch bekannt war, daß durch enzymatische Transglucanosylierung, ausgehend von Amylose, Amylopectin oder von Cyclodextrinen, substituierte α-D-maltodextrine hergestellt werden können, wobei α-Methyl- oder α-Phenyl-Glukoside als Akzeptoren eingesetzt wurden, vgl. FRENCH et al., J.Am.Chem.Soc., Band 76, 2387-2390 (1954), so ist es doch überraschend, daß als Rezeptoren hierbei nicht nur relativ einfach gebaute Glukoside wie α-Methyl- oder α-Phenylderivate eingesetzt werden können, sondern auch solche Glukoside, die als Aglukon eine Indikatorverbindung besitzen, die analytische Bedeutung bei der Bestimmung des Amylaseenzyms aufweist.

Bei einer bevorzugten Ausführungsform des Herstellungsverfahrens für die neuen Indoxylmaltodextrine wird das α-Cyclodextrin und das Indoxylglukosid in wässriger gepufferter Lösung in einer durch die Löslichkeit der Komponenten bestimmten Menge bei einem pH-Bereich von 4,0 bis 9,0, vorzugsweise von 5,5 bis 7,5, mit der Transglykanosylase bei einer Temperatur von 15° bis 45°C 2 bis 200 Stunden, vorzugsweise 30 bis 50 Stunden, z.B. für etwa 48 Stunden bei Zimmertemperatur inkubiert. Pro Mol α-Cyclodextrin sollen 0,2 bis 2 Mol, vorzugsweise etwa 1 Mol, Indoxylglukosid und 50 bis 500 mg Transglukanosylase mit einer Wirksamkeit von 45 Einheiten je mg Präparat eingesetzt werden. Nach der Inkubierung wird das Enzym inaktiviert, z.B. durch 15 Minuten langes Erhitzen der Lösung auf eine für die Inaktivierung des Enzyms ausreichende Temperatur wie beispielsweise 70° bis 90°C, vorzugsweise 85°C.
Die Indoxylmaltodextrine werden dann isoliert, wobei eine nach Kettenlänge ausgerichtete Reinigung des Produktes erfolgen soll. Zweckmäßig wird hier eines der Verfahren eingesetzt, welches ermöglicht, Saccharide verschiedenen Molekulargewichts zu trennen.

Besonders geeignet hierfür ist die Chromatographie, vor allem eine Hochdrucksäulenchromatographie. Durch eine saubere präparative Auftrennung des Umsetzungsproduktes können die gewünschten Indoxylmaltodextrine als Fraktionen, z.B. das Indoxylmaltotriosid und -maltotetraosid, ferner die höheren Homologen der Reihe bis zu 12 Glucoseresten oder mehr, gewonnen und von dem im Ansatz vorhandenen freien Indoxyl, nicht umgesetzten Glukosid sowie entstandener Glucose, Maltose und höheren Maltosiden, abgetrennt werden.

Besonders vorteilhaft für die analytische Bestimmung der Amylase sind die Indoxylmaltodextrine mit einer Kettenlänge von 4 - 6 Glucoseresten, da diese besonders schnell gespalten werden. Um deren Ausbeute beim erfindungsgemäßen Verfahren zu steigern, ist es zweckmäßig, die höheren Glieder der homologen Reihe zu den gewünschten niederen abzubauen, was dadurch geschehen kann, daß man im Anschluß an die Transglukanosylierung den Reaktionsansatz mit einer $\alpha$-Glukan-Phosphorylase behandelt.

Bevorzugt wird hierbei ein Enzym aus Klebsiella pneumoniae verwendet, wie es von LINDER, KURZ, BENDER, WALLENFELS, Eur. J. Biochem., And 70, 291 - 303 (1976), beschrieben wurde. Bei dieser Inkubation werden dann annähernd alle höheren Homologen der genannten Reihe, im wesentlichen bis zu dem Maltotetraosid abgebaut, so daß hiermit die Ausbeute der für die Analytik besonders interessanten Verbindungen wesentlich erhöht werden kann. Die Inkubation mit der $\alpha$-Glukan-Phosphorylase wird bei einem pH-Wert von 5,5 bis 7,5 durchgeführt. Zweckmäßig wird hierfür ein Phosphatpuffer der Molarität von $\frac{m}{20}$ bis $\frac{m}{10}$ verwendet. Es wurde festgestellt, daß bei Verwendung von Arsenatpuffer der Molarität von $\frac{m}{50}$ bis $\frac{m}{100}$ und Muskelphosphorylase, im wesentlichen das Indoxylmaltotetraosid entsteht. Auch hydrolytische

Enzyme können für den genannten Zweck Verwendung finden, sofern sie nur die glykosydischen Bindungen im peripheren Bereich der Maltodextrine spalten.

Das beschriebene Verfahren zur Herstellung der Indoxyl-α-D-maltodextrine ist sehr allgemein anwendbar. Der Transfer von Glukanosylresten auf glykosidische Akzeptoren hängt weder von der Natur des Aglykons noch von der Konfiguration der glykosidischen Bindung, mit welcher dieser mit der Glucose verbunden ist, ab. So kann z.B. Indoxyl-ß-D-glukosid für die Herstellung einer vergleichbaren Serie wie mit dem α-Anomeren erhalten werden.

Ebenso geeignet für die enzymatische Synthese von Indoxylmaltodextrinen sind Indoxyl-Derivat-glucoside, wie z.B. das 5-Brom-4-chlorindoxyl-glucosid, 5-Methylindoxylglucosid, 4-Äthoxyindoxylglucosid, 5-Phenylindoxylglucosid, 4-Nitroindoxylglucosid und kombinierte Derivate wie 2-Methyl-5-bromindoxylglucosid, 2-Methyl-5-phenyl-indoxylglucosid.

Die so erhaltenen Indoxylmaltodextrine eignen sich hervorragend als chromogene Substrate für die Herstellung von Schnelldiagnostika für Amylasen.

Zu diesem Zweck wird das erfindungsgemäße Substrat und an sich bekannte Verfahren zusammen mit Puffersalzen, Detergentien, Acceleratoren oder Inhibitoren (z.B. für bestimmte Isoenzyme der Amylase) sowie evtl. benötigten Hilfsenzymen, Stabilisatoren, Verdickungs- und Hydrophobierungsmitteln in wässriger Lösung auf eine saugfähige Matrix aufgebracht und dort durch schonendes Trocknen fixiert.

Als saugfähige Matrix findet hierbei im einfachsten Fall Papier Verwendung. Es können jedoch auch Kunststoffvliese oder wasseraufnehmende Filme eingesetzt werden, wobei im letzteren Fall die benötigten Chemikalien zusammen mit dem filmbildenden Material aus einer Lösung oder Suspension in Form eines Films oder einer Folie vergossen und erhärtet werden.

Die fertigen Reagenzpapiere, -vliese oder -filme werden vorteilhaft auf eine stabile Kunststoffolie aufgeklebt, die dem Testsystem eine verbesserte mechanische Stabilität gibt und eine hygienische Handhabung ermöglicht.
Die Wahl der Puffersalze ist prinzipiell unkritisch, sofern mögliche Unverträglichkeiten des jeweiligen Systems beachtet werden (z.B. Hemmung der $\alpha$-Amylase durch EDTA). Der pH-Wert liegt vorteilhafterweise in einem für Hilfsenzym und Amylase günstigen Wirkungsbereich (z.B. 5 - 8,5 für $\alpha$-Amylase und Glucosidase und 4 - 7 für ß-Amylase und Glucosidase).
Detergentien sind z.B. die bekannten nicht-ionischen Substanzen vom Typ Polyäthylenoxid und -Derivate wie z.B. Genapol(R) oder ionogene Verbindungen wie Natriumdodecylsulfat.
Als Stabilisator kann z.B. Albumin verwendet werden, als Verdickungsmittel Polyvinylpyrrolidon oder Gelatine und als Hydrophobierungsmittel Äthylcellulose.
Als Accelaratoren sind Alkalihalogenide, speziell Natriumchlorid, und als Inhibitoren sind z.B. die sehr stark wirkenden natürlichen Inhibitoren aus Getreidesamen (DT-AS 20 03 934) bekannt.

Zur Erläuterung der Erfindung wird beispielhaft die Herstellung und Anwendung der erfindungsgemäßen Substanzen beschrieben :

Beispiel 1

1.1 Herstellung der Indoxylmaltodextrine

Ein etwa molares Verhältnis von α-Cyclodextrin und Indoxylglucosid, das sind 31 g α-Cyclodextrin und 10 g Indoxylglucosid, gelöst in 1 Liter molarem 2-(N-Morpholino)-Aethansulfonsäure-Puffer vom pH 6,5, enthaltend 0,005 Mol/l Calciumchlorid, werden mit 10 mg - 450 U Transglukanosylase aus Klebsielle pneumoniae versetzt und 48 Stunden bei Zimmertemperatur gehalten. Danach wird die Lösung in 15 Minuten auf 80°C erhitzt.

Eine dünnschichtchromatographische Kontrolle zeigt, daß eine homologe Reihe indoxylierter Maltodextrine, enthaltend bis zu 12 Glukoseeinheiten in der Kette, entstanden ist.

Die Trennung der einzelnen Glieder dieser Reihe erfolgt in mehreren Chargen mit der Hochdruckflüssigkeitschromatographie in einer Säule von 1 m Länge und 3 cm Durchmesser. Als Füllmaterial wird Biogel P(R) 2, ein Copolymeres des Acrylamids und Methylenbisacrylamids, hergestellt von Bio-Rad Laboratories, Richmond, Californien, verwendet. Die Entwicklung erfolgt unter Druck mit einer pulsarmen Druckpumpe unter Verwendung von reinem, entgastem Wasser als Elutionsmittel. Als Detektor wird die parallele Schaltung eines Diffraktometers und eines Spektrophotometers mit Durchflußzellen benutzt. Das Refraktometer registriert die Konzentration aller Komponenten des Gemisches entsprechend den Konzentrationen, mit der sie von der Säule kommen, während die Photometerzelle bei einer Wellenlänge von z.B. 365 nm nur die Indikatorglukoside anzeigt. Diese werden mittels eines

Fraktionensammlers in einzelnen Gefäßen getrennt nach einzelnen Absorptionsmaxima aufgefangen.

Es wurden folgende Mengenverhältnisse der einzelnen Indoxyl-Glukoside erhalten:

| | |
|---|---|
| Indoxylmaltosid | 3,0 g |
| Indoxylmaltotriosid | 3,0 g |
| Indoxylmaltotetraosid | 3,1 g |
| Indoxylmaltopentaosid | 2,5 g |

Höhere Homologe werden in geringen Mengen gebildet (bis zum Indoxylmaltododecaosid etwa 6 g).

Bei anderen Mengenverhältnissen ergeben sich keine wesentlichen Verschiebungen in den prozentualen Anteilen der einzelnen Indoxylmaltodextrine.

1.2 Herstellung eines Teststreifens zum Nachweis von α-Amylase

o,1 $m^2$ Indikatorrohpapier (150 $g/m^2$) werden mit folgender Lösung getränkt und anschließend getrocknet:
1 l Triäthanolamin-Puffer, pH 7, 50 mmol/l, 10 g Indoxyl-α-maltotetraosid und 20.000 Units α-Glucosidase.
Das trockene Reagenzpapier wird feuchtigkeits- und lichtgeschützt gelagert.

Beispiel 2

Analog Beispiel 1.2 wird anstelle von 10 g Indoxyl-α-maltotetraosid 13 g 5-Brom-4-chlorindoxyl-α-maltopentaosid eingesetzt.

Beispiel 3

0,1 $m^2$ Indikatorrohpapier (150 $g/m^2$) werden nacheinander mit folgenden Lösungen getränkt und anschließend getrocknet.

Lösung A 1 l Citrat-Puffer, pH 6, 30 mmol/l, enthaltend
10 g Indoxyl-ß-maltotetraosid und
250.000 Units ß-Glucosidase

Lösung B 1 l Citratpuffer, pH 6, 30 mmol/l, enthaltend
20.000 Units α-Glucosidase

Mit Testpapieren dieser Art lassen sich pathologische Amylasekonzentrationen im Urin nach 5 - 8 min durch eine deutliche sichtbare Grün-Blau-Färbung nachweisen. Durch Zusatz der bereits erwähnten Begleitsubstanzen (Acceleratoren, Inhibitoren, Detergentien, Verdickungs- und Hydrophobierungsmittel sowie Stabilisatoren) läßt sich ein bezüglich Nachweisempfindlichkeit, Flüssigkeitsaufnahme (-geschwindigkeit) und Haltbarkeit modifiziertes Testpapier erhalten.

Patentansprüche :

1. Indoxylmaltodextrine, gekennzeichnet durch die allgemeine Formel :

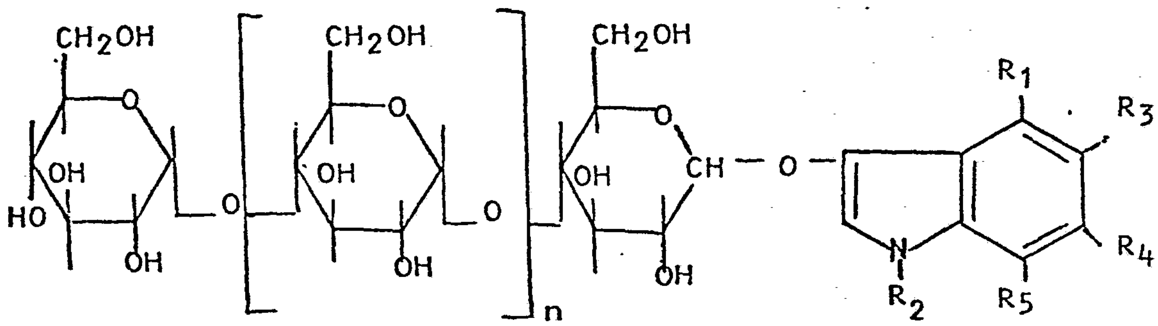

in der n eine ganze Zahl von 1 bis 9, $R_1$, $R_3$, $R_4$ und $R_5$ Halogen, Alkyl oder Alkoxyl mit 1 - 5 Kohlenstoffatomen, Phenyl, substituiertes Phenyl, Phenoxy, substituiertes Phenoxy, Nitro oder Wasserstoff, $R_2$ = H, niedriges Alkyl oder niedriges Alkoxy mit 1 - 3 C-Atomen in der Alkylkette ist und die heterosidische Glucosidbindung α- oder ß-Konfiguartion aufweist.

2. Indoxylmaltodextrine gemäß Anspruch 1, dadurch gekennzeichnet, daß n eine ganze Zahl von 2 bis 4 und R Wasserstoff ist.

3. Verfahren zur Herstellung von Indoxyl-α- oder ß-maltodextrinen nach Anspruch 1 und 2, dadurch gekennzeichnet, daß man eine wässrige Lösung von α-, ß- oder γ-Cyclodextrin und ein die Indoxylgruppe nach Anspruch 1 enthaltendes α- oder ß-D-Glucosid in Gegenwart einer Transglucanylase reagieren läßt, worauf man die synthetisierten Indoxyl-α- bzw. β-maltodextrine in an sich bekannter Weise isoliert.

4. Verfahren zur Herstellung von Indoxyl-α- oder β-maltodextrinen nach Anspruch 1 und 2, dadurch gekennzeichnet, daß man α-Maltosylfluorid und α-Amylase mit Indoxylglucosid umsetzt und die Reaktionsprodukte in an sich bekannter Weise abtrennt.

5. Verfahren nach Anspruch 3 und 4, dadurch gekennzeichnet, daß die Transglucanylase aus Klebsiella pneumoniae, Bacillus macerans oder Bacillus megatherium, die α-Amylase aus Säugetierpankreas oder -speichel isoliert wurde.

6. Verfahren nach einer der Ansprüche 3 - 5, dadurch gekennzeichnet, daß man die Reaktion in einem pH-Bereich von 4,0 - 9,0 und einer Temperatur von 15 - 45°C durchführt und sie nach 2 - 200 Stunden durch Erhitzen abbricht.

7. Verwendung einer Indoxyl-α- bzw. β-maltodextrine nach Anspruch 1 und 2 als Substrat zum Nachweis und zur quantitativen Bestimmung von Amylasen.

8. Verwendung nach Anspruch 7, dadurch gekennzeichnet, daß der Nachweis und die Bestimmung in Gegenwart von α- oder/und ß-Glucosidasen als Hilfsenzym durchgeführt wird.

9. Verwendung nach Anspruch 7 und 8, dadurch gekennzeichnet, daß der Nachweis und die Bestimmung in Gegenwart eines geeigneten Oxidationsmittels für die Oxidation des Indoxylrestes zum Indigofarbstoff durchgeführt wird.

10. Verwendung nach Anspruch 7 und 8, dadurch gekennzeichnet, daß der Nachweis und die Bestimmung in Gegenwart von Sauerstoff und einer elektronenübertragenden Verbindung durchgeführt wird.

11. Verwendung nach Anspruch 7 und 8, dadurch gekennzeichnet, daß der Nachweis und die Bestimmung in Gegenwart von $H_2O_2$ und einem dieses spaltenden Katalysators durchgeführt wird.

12. Verwendung eines Indoxylmaltodextrins nach Anspruch 1 in einem Schnelldiagnostikum zum Nachweis von Amylase und wässrigen Flüssigkeiten gekennzeichnet durch einen Gehalt des Indoxylmaltodextrins auf einem Trägermaterial gegebenenfalls zusammen mit weiteren hierfür üblichen Zusätzen.

Patentansprüche für Österreich :

1. Verfahren zur Herstellung eines Indoxylmaltodextrins der allgemeinen Formel I

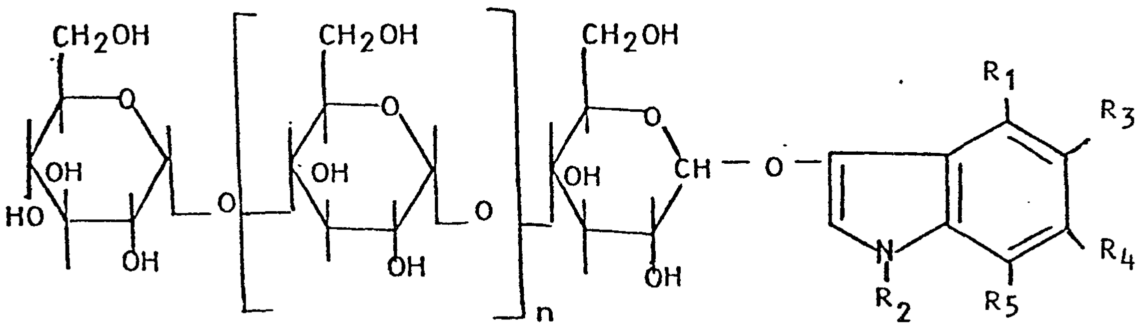

I

in der n eine ganze Zahl von 1 bis 9, $R_1$, $R_3$, $R_4$ und $R_5$ Halogen, Alkyl oder Alkoxy mit 1 - 5 Kohlenstoffatomen, Phenyl, substituiertes Phenyl, Phenoxy, substituiertes Phenoxy, Nitro oder Wasserstoff, $R_2$ = H, niedriges Alkyl oder niedriges Alkoxy mit 1 - 3 C-Atomen in der Alkylkette ist und die heterosidische Glucosidbindung α- oder ß-Konfiguration aufweist, dadurch gekennzeichnet, daß man eine wässrige Lösung von α-, ß- oder γ-Cyclodextrin und ein die Indoxylgruppe nach Formel I enthaltendes α- oder ß-D-Glucosid in Gegenwart einer Transglucanylase reagieren läßt oder α-Maltosylfluorid und α-Amylase mit Indoxylglucosid umsetzt und die Reaktionsprodukte in an sich bekannter Weise abtrennt.

2. Verfahren gemäß Anspruch 1, dadurch gekennzeichnet, daß n eine ganze Zahl von 2 bis 4 und R Wasserstoff ist.

3. Verfahren nach Anspruch 1 oder 2, dadurch gekennzeichnet, daß die Transglucanylase aus Klebsiella pneumoniae, Bacillus macerans oder Bacillus megatherium isoliert wurde.

4. Verfahren nach Anspruch 1 oder 2, dadurch gekennzeichnet, daß die α-Amylase aus Säugetierpankreas oder Speichel isoliert wurde.

5. Verwendung eines Indoxyl-α-bzw. β-maltodextrins nach Anspruch 1, 2, 3 oder 4 als Substrat zum Nachweis und zur quantitativen Bestimmung von Amylasen.

6. Verwendung eines Indoxylmaltodextrins nach Anspruch 1, 2, 3 oder 4 in einem Schnelldiagnostikum zum Nachweis von Amylase und wässrigen Flüssigkeiten, gekennzeichnet durch einen Gehalt des Indoxylmaltodextrins auf einem Trägermaterial gegebenenfalls zusammen mit weiteren hierfür üblichen Zusätzen.

0031941

Europäisches Patentamt

# EUROPÄISCHER RECHERCHENBERICHT

Nummer der Anmeldung
EP 80 10 8105

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 2 752 501 (WALLENFELS)<br>* Seiten 1-2; Patentansprüche *<br>-- | 1,5 |
| D | JOURNAL OF MEDICINAL CHEMISTRY, Band 7, No. 4, Juli 1964 Mack Printing Co. PENNSYLVANIA (US) J.P. HORWITZ et al.:"Substrates for Cytochemical Demonstration of Enzyme Activity. I. Some Substituted 3-Indolyl-β-D-glycopyranosides" Seiten 574-575<br>* Seite 574 *<br>------ | 1,5 |

| KLASSIFIKATION DER ANMELDUNG (Int Cl.³) |
|---|
| C 07 H 17/02<br>C 12 Q 1/40<br>C 12 P 19/04<br>19/26 |

| RECHERCHIERTE SACHGEBIETE (Int. Cl.³) |
|---|
| C 07 H 17/02<br>C 12 Q 1/40 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 26-03-1981 | VERHULST |